(19) Europäisches Patentamt

European Patent Office

Office européen des brevets

(11) **EP 1 533 618 A1**

(12) **EUROPEAN PATENT APPLICATION**

(43) Date of publication:
25.05.2005 Bulletin 2005/21

(51) Int Cl.7: **G01N 33/574**, C12Q 1/68

(21) Application number: **03025338.9**

(22) Date of filing: **04.11.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(71) Applicants:
- **Ludwig-Maximilians-Universität München**
  **80539 München (DE)**
  Designated Contracting States:
  **DE**
- **Roche Diagnostics GmbH**
  **68305 Mannheim (DE)**
  Designated Contracting States:
  **DE**
- **F. Hoffmann-La Roche AG**
  **4070 Basel (CH)**
  Designated Contracting States:
  **IE FR SE FI EE GB LI IT LU CH AT ES SI CZ DK SK RO GR TR PT CY MC HU BG NL BE**

(72) Inventors:
- **Dugas, Martin**
  **81375 München (DE)**
- **Schoch, Claudia**
  **81477 München (DE)**
- **Kohlmann, Alexander**
  **92318 Neumarkt (DE)**
- **Schnittger, Susanne**
  **81375 ünchen (DE)**
- **Kern, Wolfgang**
  **82319 Starnberg (DE)**
- **Haferlach, Torsten**
  **81477 München (DE)**

(74) Representative:
**Bösl, Raphael, Dr. rer. nat., Dipl.-Chem.**
**Patentanwälte**
**Isenbruck Bösl Hörschler Wichmann Huhn**
**Postfach 860 880**
**81635 München (DE)**

(54) **Method for distinguishing prognostically definable AML**

(57) Disclosed is a method for distinguishing prognostically definable AML subtypes with normal karyotype into different prognosis subsets in a sample by determining the expression level of markers, as well as a diagnostic kit and an apparatus containing the markers.

**Description**

The present invention is directed to a method for distinguishing prognostically definable AML subtypes with normal karyotypes, so far defined as prognostically intermediate, by determining the expression level of selected marker genes.

Leukemias are classified into four different groups or types: acute myeloid (AML), acute lymphatic (ALL), chronic myeloid (CML) and chronic lymphatic leukemia (CLL). Within these groups, several subcategories can be identified further using a panel of standard techniques as described below. These different subcatgories in leukemias are associated with varying clinical outcome and therefore are the basis for different treatment strategies. The importance of highly specific classification may be illustrated in detail further for the AML as a very heterogeneous group of diseases. Effort is aimed at identifying biological entities and to distinguish and classify subgroups of AML which are associated with a favorable, intermediate or unfavorable prognosis, respectively. In 1976, the FAB classification was proposed by the French-American-British co-operative group which was based on cytomorphology and cytochemistry in order to separate AML subgroups according to the morphological appearance of blasts in the blood and bone marrow. In addition, it was recognized that genetic abnormalities occurring in the leukemic blast had a major impact on the morphological picture and even more on the prognosis. So far, the karyotype of the leukemic blasts is the most important independent prognostic factor regarding response to therapy as well as survival.

Usually, a combination of methods is necessary to obtain the most important information in leukemia diagnostics: Analysis of the morphology and cytochemistry of bone marrow blasts and peripheral blood cells is necessary to establish the diagnosis. In some cases the addition of immunophenotyping is mandatory to separate very undifferentiated AML from acute lymphoblastic leukemia and CLL. Leukemia subtypes investigated can be diagnosed by cytomorphology alone, only if an expert reviews the smears. However, a genetic analysis based on chromosome analysis, fluorescence in situ hybridization or RT-PCR and immunophenotyping is required in order to assign all cases in to the right category. The aim of these techniques besides diagnosis is mainly to determine the prognosis of the leukemia. A major disadvantage of these methods, however, is that viable cells are necessary as the cells for genetic analysis have to divide in vitro in order to obtain metaphases for the analysis. Another problem is the long time of 72 hours from receipt of the material in the laboratory to obtain the result. Furthermore, great experience in preparation of chromosomes and even more in analyzing the karyotypes is required to obtain the correct result in at least 90% of cases. Using these techniques in combination, hematological malignances in a first approach are separated into chronic myeloid leukemia (CML), chronic lymphoid (CLL), acute lymphoblastic (ALL), and acute myeloid leukemia (AML). Within the latter three disease entities several prognostically relevant subtypes have been established. As a second approach this further sub-classification is based mainly on genetic abnormalities of the leukemic blasts and clearly is associated with different prognoses.

The sub-classification of leukemias becomes increasingly important to guide therapy. The development of new, specific drugs and treatment approaches requires the identification of specific subtypes that may benefit from a distinct therapeutic protocol and, thus, can improve outcome of distinct subsets of leukemia. For example, the new therapeutic drug (STI571) inhibits the CML specific chimeric tyrosine kinase BCR-ABL generated from the genetic defect observed in CML, the BCR-ABL-rearrangement due to the translocation between chromosomes 9 and 22 (t(9; 22) (q34; q11)). In patients treated with this new drug, the therapy response is dramatically higher as compared to all other drugs that had been used so far. Another example is the subtype of acute myeloid leukemia AML M3 and its variant M3v both with karyotype t[15;17](q22; q11-12). The introduction of a new drug (all-trans retinoic acid - ATRA) has improved the outcome in this subgroup of patient from about 50% to 85 % long-term survivors. As it is mandatory for these patients suffering from these specific leukemia subtypes to be identified as fast as possible so that the best therapy can be applied, diagnostics today must accomplish sub-classification with maximal precision. Not only for these subtypes but also for several other leukemia subtypes different treatment approaches could improve outcome. Therefore, rapid and precise identification of distinct leukemia subtypes is the future goal for diagnostics.

Thus, the technical problem underlying the present invention was to provide means for leukemia diagnostics which overcome at least some of the disadvantages of the prior art diagnostic methods, in particular encompassing the time-consuming and unreliable combination of different methods and which provides a rapid assay to unambigously distinguish one AML subtype from another, e.g. by genetic analysis.

According to Golub et al. (Science, 1999, 286, 531-7), gene expression profiles can be used for class prediction and discriminating AML from ALL samples. However, for the analysis of acute leukemias the selection of the two different subgroups was performed using exclusively morphologic-phenotypical criteria. This was only descriptive and does not provide deeper insights into the pathogenesis or the underlying biology of the leukemia. The approach reproduces only very basic knowledge of cytomorphology and intends to differentiate classes. The data is not sufficient to predict prognostically relevant cytogenetic aberrations.

Furthermore, the international application WO-A 03/039443 discloses marker genes the expression levels of which are characteristic for certain leukemia, e.g. AML subtypes and additionally discloses methods for differentiating between the subtype of AML cells by determining the expression profile of the disclosed marker genes. However, WO-A 03/039443 does not provide guidance which set of distinct genes discriminate between two subtypes and, as such, can be routineously taken in order to distinguish one AML subtype from another.

The problem is solved by the present invention, which provides a method for distinguishing prognostically definable AML subtypes with normal karyotype into different prognosis subsets in a sample, the method comprising determining the expression level of markers selected from the markers identifiable by their Affymetrix Identification Numbers (affy id) as defined in Table 1,

wherein

a high expression of at least one polynucleotide defined by any of the numbers 1, 2, 3, 4, 5, 6, 7, 8, 9, 10, 11, 12, 13, 14, 15, 16, 17, 19, 20, 21, 22, 23, 24, 25, 26, 27, 28, 29, 30, 31, 32, 33, 34, 35, 36, 37, 38, 39, 40, 42, 43, 44, 46, 47, 48, 49, and/or 50 of Table 1,

is indicative for a specific median event-free survival (EFS).

According to the present invention, a "sample" means any biological material containing genetic information in the form of nucleic acids or proteins obtainable or obtained from an individual. The sample includes e.g. tissue samples, cell samples, bone marrow and/or body fluids such as blood, saliva, semen. Preferably, the sample is blood or bone marrow, more preferably the sample is bone marrow. The person skilled in the art is aware of methods, how to isolate nucleic acids and proteins from a sample. A general method for isolating and preparing nucleic acids from a sample is outlined in Example 3.

According to the present invention, the term "lower expression" is generally assigned to all by numbers and Affymetrix Id. definable polynucleotides the t-values and fold change (fc) values of which are negative, as indicated in the Tables. Accordingly, the term "higher expression" is generally assigned to all by numbers and Affymetrix Id. definable polynucleotides the t-values and fold change (fc) values of which are positive.

According to the present invention, the term "expression" refers to the process by which mRNA or a polypeptide is produced based on the nucleic acid sequence of a gene, i.e. "expression" also includes the formation of mRNA upon transcription. In accordance with the present invention, the term "determining the expression level" preferably refers to the determination of the level of expression, namely of the markers.

Generally, "marker" refers to any genetically controlled difference which can be used in the genetic analysis of a test versus a control sample, for the purpose of assigning the sample to a defined genotype or phenotype. As used herein, "markers" refer to genes which are differentially expressed in, e.g., different AML subtypes. The markers can be defined by their gene symbol name, their encoded protein name, their transcript identification number (cluster identification number), the data base accession number, public accession number or GenBank identifier or, as done in the present invention, Affymetrix identification number, chromosomal location, UniGene accession number and cluster type, LocusLink accession number (see Examples and Tables).

The Affymetrix identification number (affy id) is accessible for anyone and the person skilled in the art by entering the "gene expression omnibus" internet page of the National Center for Biotechnology Information (NCBI) (http://www.ncbi.nlm.nih.gov/geo/). In particular, the affy id's of the polynucleotides used for the method of the present invention are derived from the so-called U133 chip. The sequence data of each identification number can be viewed at http://www.ncbi.nlm.nih.gov/geo/query/acc.cgi?acc=GPL96

Generally, the expression level of a marker is determined by the determining the expression of its corresponding "polynucleotide" as described hereinafter.

According to the present invention, the term "polynucleotide" refers, generally, to a DNA, in particular cDNA, or RNA, in particular a cRNA, or a portion thereof or a polypeptide or a portion thereof. In the case of RNA (or cDNA), the polynucleotide is formed upon transcription of a nucleotide sequence which is capable of expression. The polynucleotide fragments refer to fragments preferably of between at least 8, such as 10, 12, 15 or 18 nucleotides and at least 50, such as 60, 80, 100, 200 or 300 nucleotides in length, or a complementary sequence thereto, representing a consecutive stretch of nucleotides of a gene, cDNA or mRNA. In other terms, polynucleotides include also any fragment (or complementary sequence thereto) of a sequence derived from any of the markers defined above as long as these fragments unambiguously identify the marker.

The determination of the expression level may be effected at the transcriptional or translational level, i.e. at the level of mRNA or at the protein level. Protein fragments such as peptides or polypeptides advantageously comprise between at least 6 and at least 25, such as 30, 40, 80, 100 or 200 consecutive amino acids representative of the corresponding full length protein. Six amino acids are generally recognized as the lowest peptidic stretch giving rise to a linear epitope recognized by an antibody, fragment or derivative thereof. Alternatively, the proteins or fragments thereof may be analysed using nucleic acid molecules specifically binding to three-dimensional structures (aptamers).

Depending on the nature of the polynucleotide or polypeptide, the determination of the expression levels may be effected by a variety of methods. For determining and detecting the expression level, it is preferred in the present invention that the polynucleotide, in particular the cRNA, is labelled.

The labelling of the polynucleotide or a polypeptide can occur by a variety of methods known to the skilled artisan. The label can be fluorescent, chemiluminescent, bioluminescent, radioactive (such as $^3$H or $^{32}$P). The labelling compound can be any labelling compound being suitable for the labelling of polynucleotides and/or polypeptides. Examples include fluorescent dyes, such as fluorescein, dichlorofluorescein, hexachlorofluorescein, BODIPY variants, ROX, tetramethylrhodamin, rhodamin X, Cyanine-2, Cyanine-3, Cyanine-5, Cyanine-7, IRD40, FluorX, Oregon Green, Alexa variants (available e.g. from Molecular Probes or Amersham Biosciences) and the like, biotin or biotinylated nucleotides, digoxigenin, radioisotopes, antibodies, enzymes and receptors. Depending on the type of labelling, the detection is done via fluorescence measurements, conjugation to streptavidin and/or avidin, antigen-antibody- and/or antibody-antibody-interactions, radioactivity measurements, as well as catalytic and/or receptor/ligand interactions. Suitable methods include the direct labelling (incorporation) method, the amino-modified (amino-allyl) nucleotide method (available e.g. from Ambion), and the primer tagging method (DNA dendrimer labelling, as kit available e.g. from Genisphere). Particularly preferred for the present invention is the use of biotin or biotinylated nucleotides for labelling, with the latter being directly incorporated into, e.g. the cRNA polynucleotide by in vitro transcription.

If the polynucleotide is mRNA, cDNA may be prepared into which a detectable label, as exemplified above, is incorporated. Said detectably labelled cDNA, in single-stranded form, may then be hybridised, preferably under stringent or highly stringent conditions to a panel of single-stranded oligonucleotides representing different genes and affixed to a solid support such as a chip. Upon applying appropriate washing steps, those cDNAs will be detected or quantitatively detected that have a counterpart in the oligonucleotide panel. Various advantageous embodiments of this general method are feasible. For example, the mRNA or the cDNA may be amplified e.g. by polymerase chain reaction, wherein it is preferable, for quantitative assessments, that the number of amplified copies corresponds relative to further amplified mRNAs or cDNAs to the number of mRNAs originally present in the cell. In a preferred embodiment of the present in ivention, the cDNAs are transcribed into cRNAs prior to the hybridisation step wherein only in the transcription step a label is incorporated into the nucleic acid and wherein the cRNA is employed for hybridisation. Alternatively, the label may be attached subsequent to the transcription step.

Similarly, proteins from a cell or tissue under investigation may be contacted with a panel of aptamers or of antibodies or fragments or derivatives thereof. The antibodies etc. may be affixed to a solid support such as a chip. Binding of proteins indicative of an AML subtype may be verified by binding to a detectably labelled secondary antibody or aptamer. For the labelling of antibodies, it is referred to Harlow and Lane, "Antibodies, a laboratory manual", CSH Press, 1988, Cold Spring Harbor. Specifically, a minimum set of proteins necessary for diagnosis of all AML subtypes may be selected for creation of a protein array system to make diagnosis on a protein lysate of a diagnostic bone marrow sample directly. Protein Array Systems for the detection of specific protein expression profiles already are available (for example: Bio-Plex, BIORAD, München, Germany). For this application preferably antibodies against the proteins have to be produced and immobilized on a platform e.g. glasslides or microtiterplates. The immobilized antibodies can be labelled with a reactant specific for the certain target proteins as discussed above. The reactants can include enzyme substrates, DNA, receptors, antigens or antibodies to create for example a capture sandwich immunoassay.

For reliably distinguishing prognostically definable AML subtypes with normal karyotype into different prognosis subsetsit is useful that the expression of more than one of the above defined markers is determined. As a criterion for the choice of markers, the statistical significance of markers as expressed in q or p values based on the concept of the false discovery rate is determined. In doing so, a measure of statistical significance called the $q$ value is associated with each tested feature. The $q$ value is similar to the $p$ value, except it is a measure of significance in terms of the false discovery rate rather than the false positive rate (Storey JD and Tibshirani R. Proc.Natl.Acad.Sci., 2003, Vol. 100: 9440-5.

In a preferred embodiment of the present invention, markers as defined in Tables 1-7 having a p-value of less than 3E-02, more preferred less than 1.5E-04, most preferred less than 1.5E-05, less than 1.5E-06, are measured.

Of the above defined markers, the expression level of at least two, preferably of at least ten, more preferably of at least 25, most preferably of 50 of the Table of the markers is determined.

In another preferred embodiment, the expression level of at least 2, of at least 5, of at least 10 out of the markers having the numbers 1 - 10, 1-20, 1-40, 1-50 of the Table are measured.

The level of the expression of the "marker", i.e. the expression of the polynucleotide is indicative of the AML subtype of a cell or an organism. The level of expression of a marker or group of markers is measured and is compared with the level of expression of the same marker or the same group of markers from other cells or samples. The comparison may be effected in an actual experiment or in silico. When the expression level also referred to as expression pattern or expression signature (expression profile) is measurably different, there is according to the invention a meaningful difference in the level of expression. Preferably the difference at least is 5 %, 10% or 20%, more preferred at least 50% or may even be as high as 75% or 100%. More preferred the difference in the level of expression is at least 200%, i.e. two fold, at least 500%, i.e. five fold, or at least 1000%, i.e. 10 fold.

Accordingly, the expression level of markers expressed lower in a first subtype than in at least one second subtype, which differs from the first subtype, is at least 5 %, 10% or 20%, more preferred at least 50% or may even be 75% or 100%, i.e. 2-fold lower, preferably at least 10-fold, more preferably at least 50-fold, and most preferably at least 100-fold lower in the first subtype. On the other hand, the expression level of markers expressed higher in a first subtype than in at least one second subtype, which differs from the first subtype, is at least 5 %, 10% or 20%, more preferred at least 50% or may even be 75% or 100%, i.e. 2-fold higher, preferably at least 10-fold, more preferably at least 50-fold, and most preferably at least 100-fold higher in the first subtype.

In another embodiment of the present invention, the sample is derived from an individual having leukaemia, preferably AML.

For the method of the present invention it is preferred if the polynucleotide the expression level of which is determined is in form of a transcribed polynucleotide. A particularly preferred transcribed polynucleotide is an mRNA, a cDNA and/or a cRNA, with the latter being preferred. Transcribed polynucleotides are isolated from a sample, reverse transcribed and/or amplified, and labelled, by employing methods well-known the person skilled in the art (see Example 3). In a preferred embodiment of the methods according to the invention, the step of determining the expression profile further comprises amplifying the transcribed polynucleotide.

In order to determine the expression level of the transcribed polynucleotide by the method of the present invention, it is preferred that the method comprises hybridizing the transcribed polynucleotide to a complementary polynucleotide, or a portion thereof, under stringent hybridization conditions, as described hereinafter.

The term "hybridizing" means hybridization under conventional hybridization conditions, preferably under stringent conditions as described, for example, in Sambrook, J., et al., in "Molecular Cloning: A Laboratory Manual" (1989), Eds. J. Sambrook, E.F. Fritsch and T. Maniatis, Cold Spring Harbour Laboratory Press, Cold Spring Harbour, NY and the further definitions provided above. Such conditions are, for example, hybridization in 6x SSC, pH 7.0 / 0.1% SDS at about 45°C for 18-23 hours, followed by a washing step with 2x SSC/0.1% SDS at 50°C. In order to select the stringency, the salt concentration in the washing step can for example be chosen between 2x SSC/0.1% SDS at room temperature for low stringency and 0.2x SSC/0.1% SDS at 50°C for high stringency. In addition, the temperature of the washing step can be varied between room temperature, ca. 22°C, for low stringency, and 65°C to 70° C for high stringency. Also contemplated are polynucleotides that hybridize at lower stringency hybridization conditions. Changes in the stringency of hybridization and signal detection are primarily accomplished through the manipulation, preferably of formamide concentration (lower percentages of formamide result in lowered stringency), salt conditions, or temperature. For example, lower stringency conditions include an overnight incubation at 37°C in a solution comprising 6X SSPE (20X SSPE = 3M NaCl; 0.2M NaH2PO4; 0.02M EDTA, pH 7.4), 0.5% SDS, 30% formamide, 100 mg/ml salmon sperm blocking DNA, followed by washes at 50°C with 1 X SSPE, 0.1% SDS. In addition, to achieve even lower stringency, washes performed following stringent hybridization can be done at higher salt concentrations (e.g. 5x SSC). Variations in the above conditions may be accomplished through the inclusion and/or substitution of alternate blocking reagents used to suppress background in hybridization experiments. The inclusion of specific blocking reagents may require modification of the hybridization conditions described above, due to problems with compatibility.

"Complementary" and "complementarity", respectively, can be described by the percentage, i.e. proportion, of nucleotides which can form base pairs between two polynucleotide strands or within a specific region or domain of the two strands. Generally, complementary nucleotides are, according to the base pairing rules, adenine and thymine (or adenine and uracil), and cytosine and guanine. Complementarity may be partial, in which only some of the nucleic acids' bases are matched according to the base pairing rules. Or, there may be a complete or total complementarity between the nucleic acids. The degree of complementarity between nucleic acid strands has effects on the efficiency and strength of hybridization between nucleic acid strands.

Two nucleic acid strands are considered to be 100% complementary to each other over a defined length if in a defined region all adenines of a first strand can pair with a thymine (or an uracil) of a second strand, all guanines of a first strand can pair with a cytosine of a second strand, all thymine (or uracils) of a first strand can pair with an adenine of a second strand, and all cytosines of a first strand can pair with a guanine of a second strand, and vice versa. According to the present invention, the degree of complementarity is determined over a stretch of 20, preferably 25, nucleotides, i.e. a 60% complementarity means that within a region of 20 nucleotides of two nucleic acid strands 12 nucleotides of the first strand can base pair with 12 nucleotides of the second strand according to the above ruling, either as a stretch of 12 contiguous nucleotides or interspersed by non-pairing nucleotides, when the two strands are attached to each other over said region of 20 nucleotides. The degree of complementarity can range from at least about 50% to full, i.e. 100% complementarity. Two single nucleic acid strands are said to be "substantially complementary" when they are at least about 80% complementary, preferably about 90% or higher. For carrying out the method of the present invention substantial complementarity is preferred.

Preferred methods for detection and quantification of the amount of polynucleotides, i.e. for the methods according to the invention allowing the determination of the level of expression of a marker, are those described by Sambrook et al. (1989) or real time methods known in the art as the TaqMan® method disclosed in WO92/02638 and the corresponding U.S. 5,210,015, U.S. 5,804,375, U.S. 5,487,972. This method exploits the exonuclease activity of a polymerase to generate a signal. In detail, the (at least one) target nucleic acid component is detected by a process comprising contacting the sample with an oligonucleotide containing a sequence complementary to a region of the target nucleic acid component and a labeled oligonucleotide containing a sequence complementary to a second region of the same target nucleic acid component sequence strand, but not including the nucleic acid sequence defined by the first oligonucleotide, to create a mixture of duplexes during hybridization conditions, wherein the duplexes comprise the target nucleic acid annealed to the first oligonucleotide and to the labeled oligonucleotide such that the 3'-end of the first oligonucleotide is adjacent to the 5'-end of the labeled oligonucleotide. Then this mixture is treated with a template-dependent nucleic acid polymerase having a 5' to 3' nuclease activity under conditions sufficient to permit the 5' to 3' nuclease activity of the polymerase to cleave the annealed, labeled oligonucleotide and release labeled fragments. The signal generated by the hydrolysis of the labeled oligonucleotide is detected and/ or measured. Taq-Man® technology eliminates the need for a solid phase bound reaction complex to be formed and made detectable. Other methods include e.g. fluorescence resonance energy transfer between two adjacently hybridized probes as used in the LightCycler® format described in U.S. 6,174,670.

A preferred protocol if the marker, i.e. the polynucleotide, is in form of a transcribed nucleotide, is described in Example 3, where total RNA is isolated, cDNA and, subsequently, cRNA is synthesized and biotin is incorporated during the transcription reaction. The purified cRNA is applied to commercially available arrays which can be obtained e.g. from Affymetrix. The hybridized cRNA is detected according to the methods described in Example 3. The arrays are produced by photolithography or other methods known to experts skilled in the art e.g. from U.S. 5,445,934, U.S. 5,744,305, U.S. 5,700,637, U.S. 5,945,334 and EP 0 619 321 or EP 0 373 203, or as decribed hereinafter in greater detail.

In another embodiment of the present invention, the polynucleotide or at least one of the polynucleotides is in form of a polypeptide. In another preferred embodiment, the expression level of the polynucleotides or polypeptides is detected using a compound which specifically binds to the polynucleotide of the polypeptide of the present invention.

As used herein, "specifically binding" means that the compound is capable of discriminating between two or more polynucleotides or polypeptides, i.e. it binds to the desired polynucleotide or polypeptide, but essentially does not bind unspecifically to a different polynucleotide or polypeptide.

The compound can be an antibody, or a fragment thereof, an enzyme, a so-called small molecule compound, a protein-scaffold, preferably an anticalin. In a preferred embodiment, the compound specifically binding to the polynucleotide or polypeptide is an antibody, or a fragment thereof.

As used herein, an "antibody" comprises monoclonal antibodies as first described by Köhler and Milstein in Nature 278 (1975), 495-497 as well as polyclonal antibodies, i.e. entibodies contained in a polyclonal antiserum. Monoclonal antibodies include those produced by transgenic mice. Fragments of antibodies include $F(ab')_2$, Fab and Fv fragments. Derivatives of antibodies include scFvs, chimeric and humanized antibodies. See, for example Harlow and Lane, loc. cit. For the detection of polypeptides using antibodies or fragments thereof, the person skilled in the art is aware of a variety of methods, all of which are included in the present invention. Examples include immunoprecipitation, Western blotting, Enzyme-linked immuno sorbent assay (ELISA), Enzyme-linked immuno sorbent assay (RIA), dissociation-enhanced lanthanide fluoro immuno assay (DELFIA), scintillation proximity assay (SPA). For detection, it is desirable if the antibody is labelled by one of the labelling compounds and methods described supra.

In another preferred embodiment of the present invention, the method for distinguishing prognostically definable AML subtypes with normal karyotype into different prognosis subsets is carried out on an array.

In general, an "array" or "microarray" refers to a linear or two- or three dimensional arrangement of preferably discrete nucleic acid or polypeptide probes which comprises an intentionally created collection of nucleic acid or polypeptide probes of any length spotted onto a substrate/solid support. The person skilled in the art knows a collection of nucleic acids or polypeptide spotted onto a substrate/solid support also under the term "array". As known to the person skilled in the art, a microarray usually refers to a miniaturised array arrangement, with the probes being attached to a density of at least about 10, 20, 50, 100 nucleic acid molecules referring to different or the same genes per $cm^2$. Furthermore, where appropriate an array can be referred to as "gene chip". The array itself can have different formats, e.g. libraries of soluble probes or libraries of probes tethered to resin beads, silica chips, or other solid supports.

The process of array fabrication is well-known to the person skilled in the art. In the following, the process for preparing a nucleic acid array is described. Commonly, the process comprises preparing a glass (or other) slide (e.g. chemical treatment of the glass to enhance binding of the nucleic acid probes to the glass surface), obtaining DNA sequences representing genes of a genome of interest, and spotting sequences these sequences of interest onto glass slide. Sequences of interest can be obtained via creating a cDNA library from an mRNA source or by using publicly available databases, such as GeneBank, to annotate the sequence information of custom cDNA libraries or to identify cDNA clones from previously prepared libraries. Generally, it is recommendable to amplify obtained sequences by PCR in order to have sufficient amounts of DNA to print on the array. The liquid containing the amplified probes can be deposited on the array by using a set of microspotting pins. Ideally, the amount deposited should be uniform. The process can further include UV-crosslinking in order to enhance immobilization of the probes on the array.

In a preferred embodiment, the array is a high density oligonucleotide (oligo) array using a light-directed chemical synthesis process, employing the so-called photolithography technology. Unlike common cDNA arrays, oligo arrays (according to the Affymetrix technology) use a single-dye technology. Given the sequence information of the markers, the sequence can be synthesized directly onto the array, thus, bypassing the need for physical intermediates, such as PCR products, required for making cDNA arrays. For this purpose, the marker, or partial sequences thereof, can be represented by 14 to 20 features, preferably by less than 14 features, more preferably less than 10 features, even more preferably by 6 features or less, with each feature being a short sequence of nucleotides (oligonucleotide), which is a perfect match (PM) to a segment of the respective gene. The PM oligonucleotide are paired with mismatch (MM) oligonucleotides which have a single mismatch at the central base of the nucleotide and are used as "controls". The chip exposure sites are defined by masks and are deprotected by the use of light, followed by a chemical coupling step resulting in the synthesis of one nucleotide. The masking, light deprotection, and coupling process can then be repeated to synthesize the next nucleotide, until the nucleotide chain is of the specified length.

Advantageously, the method of the present invention is carried out in a robotics system including robotic plating and a robotic liquid transfer system, e.g. using microfluidics, i.e. channelled structured.

A particular preferred method according to the present invention is as follows:

1. Obtaining a sample, e.g. bone marrow or peripheral blood aliquots, from a patient having AML
2. Extracting RNA, preferably mRNA, from the sample
3. Reverse transcribing the RNA into cDNA
4. In vitro transcribing the cDNA into cRNA
5. Fragmenting the cRNA
6. Hybridizing the fragmented cRNA on standard microarrays
7. Determining hybridization

In another embodiment, the present invention is directed to the use of at least one marker selected from the markers identifiable by their Affymetrix Identification Numbers (affy id) as defined in Tables 1, and/or 2 for the manufacturing of a diagnostic for distinguishing prognostically definable AML subtypes with normal karyotype. The use of the present invention is particularly advantageous for distinguishing prognostically definable AML subtypes with normal karyotype into different prognosis subsets in an individual having AML. The use of said markers for diagnosis of prognostically definable AML subtypes with normal karyotype, preferably based on microarray technology, offers the following advantages: (1) more rapid and more precise diagnosis, (2) easy to use in laboratories without specialized experience, (3) abolishes the requirement for analyzing viable cells for chromosome analysis (transport problem), and (4) very experienced hematologists for cytomorphology and cytochemistry, immunophenotyping as well as cytogeneticists and molecularbiologists are no longer required.

Accordingly, the present invention refers to a diagnostic kit containing at least one marker selected from the markers identifiable by their Affymetrix Identification Numbers (affy id) as defined in Table 1 for distinguishing prognostically definable AML subtypes with normal karyotype, in combination with suitable auxiliaries. Suitable auxiliaries, as used herein, include buffers, enzymes, labelling compounds, and the like. In a preferred embodiment, the marker contained in the kit is a nucleic acid molecule which is capable of hybridizing to the mRNA corresponding to at least one marker of the present invention. Preferably, the at least one nucleic acid molecule is attached to a solid support, e.g. a polystyrene microtiter dish, nitrocellulose membrane, glass surface or to non-immobilized particles in solution.

In another preferred embodiment, the diagnostic kit contains at least one reference for a prognostically definable AML subtype with normal karyotype. As used herein, the reference can be a sample or a data bank.

In another embodiment, the present invention is directed to an apparatus for distinguishing prognostically definable AML subtypes with normal karyotypes, containing a reference data bank obtainable by comprising

(a) compiling a gene expression profile of a patient sample by determining the expression level at least one marker selected from the markers identifiable by their Affymetrix Identification Numbers (affy id) as defined in Table 1,and
(b) classifying the gene expression profile by means of a machine learning algorithm.

According to the present invention, the "machine learning algorithm" is a computational-based prediction methodology, also known to the person skilled in the art as "classifier", employed for characterizing a gene expression profile. The signals corresponding to a certain expression level which are obtained by the microarray hybridization are subjected to the algorithm in order to classify the expression profile. Supervised learning involves "training" a classifier to recognize the distinctions among classes and then "testing" the accuracy of the classifier on an independent test set. For new, unknown sample the classifier shall predict into which class the sample belongs.

Preferably, the machine learning algorithm is selected from the group consisting of Weighted Voting, K-Nearest Neighbors, Decision Tree Induction, Support Vector Machines (SVM), and Feed-Forward Neural Networks. Most preferably, the machine learning algorithm is Support Vector Machine, such as polynomial kernel and Gaussian Radial Basis Function-kernel SVM models.

The classification accuracy of a given gene list for a set of microarray experiments is preferably estimated using Support Vector Machines (SVM), because there is evidence that SVM-based prediction slightly outperforms other classification techniques like k-Nearest Neighbors (k-NN). The LIBSVM software package version 2.36 was used (SVM-type: C-SVC, linear kernel (http://www.csie.ntu.edu.tw/~cjlin/libsvm/)). The skilled artisan is furthermore referred to Brown et al., Proc.Natl.Acad.Sci., 2000; 97: 262-267, Furey et al., Bioinformatics. 2000; 16: 906-914, and Vapnik V. Statistical Learning Theory. New York: Wiley, 1998.

In detail, the classification accuracy of a given gene list for a set of microarray experiments can be estimated using Support Vector Machines (SVM) as supervised learning technique. Generally, SVMs are trained using differentially expressed genes which were identified on a subset of the data and then this trained model is employed to assign new samples to those trained groups from a second and different data set. Differentially expressed genes were identified applying ANOVA and t-test-statistics (Welch t-test). Based on identified distinct gene expression signatures respective training sets consisting of 2/3 of cases and test sets with 1/3 of cases to assess classification accuracies are designated. Assignment of cases to training and test set is randomized and balanced by diagnosis. Based on the training set a Support Vector Machine (SVM) model is built.

According to the present invention, the apparent accuracy, i.e. the overall rate of correct predictions of the complete data set was estimated by 10fold cross validation. This means that the data set was divided into 10 approximately equally sized subsets, an SVM-model was trained for 9 subsets and predictions were generated for the remaining subset. This training and prediction process was repeated 10 times to include predictions for each subset. Subsequently the data set was split into a training set, consisting of two thirds of the samples, and a test set with the remaining one third. Apparent accuracy for the training set was estimated by 10fold cross validation (analogous to apparent accuracy for complete set). A SVM-model of the training set was built to predict diagnosis in the independent test set, thereby estimating true accuracy of the prediction model. This prediction approach was applied both for overall classification (multi-class) and binary classification (diagnosis X => yes or no). For the latter, sensitivity and specificity were calculated:

$$\text{Sensitivity} = (\text{number of positive samples predicted})/(\text{number of true positives})$$

$$\text{Specificity} = (\text{number of negative samples predicted})/(\text{number of true negatives})$$

In a preferred embodiment, the reference data bank is backed up on a computational data memory chip which can be inserted in as well as removed from the apparatus of the present invention, e.g. like an interchangeable module, in order to use another data memory chip containing a different reference data bank.

The apparatus of the present invention containing a desired reference data bank can be used in a way such that an unknown sample is, first, subjected to gene expression profiling, e.g. by microarray analysis in a manner as described supra or in the art, and the expression level data obtained by the analysis are, second, fed into the apparatus and compared with the data of the reference data bank obtainable by the above method. For this purpose, the apparatus suitably contains a device for entering the expression level of the data, for example a control panel such as a keyboard. The results, whether and how the data of the unknown sample fit into the reference data bank can be made visible on a provided monitor or display screen and, if desired, printed out on an incorporated of connected printer.

Alternatively, the apparatus of the present invention is equipped with particular appliances suitable for detecting and measuring the expression profile data and, subsequently, proceeding with the comparison with the reference data bank. In this embodiment, the apparatus of the present invention can contain a gripper arm and/or a tray which takes up the microarray containing the hybridized nucleic acids.

In another embodiment, the present invention refers to a reference data bank for distinguishing prognostically definable AML subtypes with normal karyotypes in a sample obtainable by comprising

> (a) compiling a gene expression profile of a patient sample by determining the expression level of at least one marker selected from the markers identifiable by their Affymetrix Identification Numbers (affy id) as defined in Table 1, and
> (b) classifying the gene expression profile by means of a machine learning algorithm.

Preferably, the reference data bank is backed up and/or contained in a computational memory data chip.

The invention is further illustrated in the following Table and Examples, without limiting the scope of the invention:

TABLE 1

Table 1 show AML subtype analysis of prognostically definable AML subtypes with normal karyotypes. The analysed markers are ordered according to their p-values, beginning with the lowest p-values.
For convenience and a better understanding, Table 1 is accompanied with explanatory tables (Table 1A) where the numbering and the Affymetrix Id are further defined by other parameters, e.g. gene bank accession number.

**EXAMPLES**

**Example 1: General experimental design of the invention and results**

Prognostication in acute myeloid leukemia with normal karyotype (AML-NK) is needed desperately. Identified molecular genetic markers like length mutations of the FLT3 gene and internal tandem duplications of the MLL gene are considered the first steps in this direction. We determined gene expression profiles using U133A+B microarrays (Affymetrix) in 143 patients with AML-NK to obtain genetic markers useful for the estimation of the prognosis of these patients. All patients were treated by standard induction and post-remission therapies. Univariate Cox regression analyses were performed to detect relations between the expression status of more than 30,000 genes and EFS in the training cohort (TRC) comprizing 85 patients with AML-NK (median age 58 years, range 17-82; median event-free survival (EFS) 7.2 months, median overall survival (OS) 9.1 months, median relapse-free survival (RFS) 11.3 months,). The 50 top genes revealing the most significant relation to EFS were then analyzed for relation to EFS in the test cohort (TEC) comprizing 58 additional patients with AML-NK. Out of these 50 top genes two had impact on EFS in TEC (TRIM16, p=0.001 for TRC and p=0.055 for TEC; TM4SF1, p=0.001 for TRC and p=0.049 for TEC). Grouping the patients according to the expression status of these two genes (0 or 1 of 2 vs. 2 of 2 expressed) resulted in significant differences of median EFS: 9.4 vs. 5.9 months (33% vs. 0% at 2 years, p=0.0012, n=58 vs. 27) for TRC and 9.8 vs. 3.2 (n=38 vs. 20, p=0.0592) for TEC. Similar differences were observed in median relapse-free survival (RFS; 20.9 vs. 5.5 months, 47% vs. 0% at 2 years, p=0.0003 for TRC; 9.0 vs. 2.8 months, n.s. for TEC) and in median overall survival (OS; 10.7 vs. 7.0 months, 45% vs. 20% at 2 years, p=0.0181 for TRC; 15.5 vs. 14.0 months, n.s. for TEC). TRIM 16 (located at 17p 11.2) encodes for a new member of a subfamily within the B box zinc finger protein family, including transcription factors. TM4SF1 (located at 3q21-q25) encodes for a plasma membrane protein. Members of the TM4 super family have been implicated in the regulation of cancer cell metastases and were shown to confer an unfavorable prognostic impact in cancer patients. Thus, both genes identified in the present analyses may be considered candidates causing a more aggressive type of AML which is in line with the observed unfavorable prognostic impact. As has been demonstrated by Yeoh et al. (Cancer Cell 2002) prognostication is possible only by combining the expression data of several genes even in biologically homogeneous subtypes of leukemia, e.g. of childhood acute lymphoblastic leukemia. Consequently, the limited number of prognostic genes in two independent cohorts of patients in the present analysis suggests a high degree of heterogeneity in AML-NK. Therefore, the definition of genetically defined parameters useful for prognostication may be best put into practice in biologically homogeneous leukemia subtypes like the CBF leukemias. In contrast, in AML-NK prognostication may at present be accomplished best by using phenotypic and treatment-dependent parameters like burden of disease at diagnosis and minimal residual disease during complete remission.

**Example 2: General materials, methods and definitions of functional annotations**

The methods section contains both information on statistical analyses used for identification of differentially expressed genes and detailed annotation data of identified microarray probesets.

Affymetrix Probeset Annotation

All annotation data of GeneChip® arrays are extracted from the NetAffx™ Analysis Center (internet website: www.affymetrix.com). Files for U133 set arrays, including U133A and U133B microarrays are derived from the June 2003 release. The original publication refers to: Liu G, Loraine AE, Shigeta R, Cline M, Cheng J, Valmeekam V, Sun S, Kulp D, Siani-Rose MA. NetAffx: Affymetrix probesets and annotations. Nucleic Acids Res. 2003;31(1):82-6.
The sequence data are omitted due to their large size, and because they do not change, whereas the annotation data are updated periodically, for example new information on chromomal location and functional annotation of the respective gene products. Sequence data are available for download in the NetAffx Download Center (www.affymetrix.com)

Data fields:

In the following section, the content of each field of the data files are described. Microarray probesets, for example found to be differentially expressed between different types of leukemia samples are further described by additional information. The fields are of the following types:

    1. GeneChip Array Information
    2. Probe Design Information
    3. Public Domain and Genomic References

1. GeneChip Array Information

HG-U133 Probeset_ID:

HG-U133 ProbeSet_ID describes the probe set identifier. Examples are: 200007_at, 200011_s_at, 200012_x_at.

GeneChip:

The description of the GeneChip probe array name where the respective probeset is represented. Examples are: Affymetrix Human Genome U133A Array or Affymetrix Human Genome U133B Array.

2. Probe Design Information

Sequence Type:

The Sequence Type indicates whether the sequence is an Exemplar, Consensus or Control sequence. An Exemplar is a single nucleotide sequence taken directly from a public database. This sequence could be an mRNA or EST. A Consensus sequence, is a nucleotide sequence assembled by Affymetrix, based on one or more sequence taken from a public database.

Transcript ID:

The cluster identification number with a sub-cluster identifier appended.

Sequence Derived From:

The accession number of the single sequence, or representative sequence on which the probe set is based. Refer to the "Sequence Source" field to determine the database used.

Sequence ID:

For Exemplar sequences: Public accession number or GenBank identifier. For Consensus sequences: Affymetrix identification number or public accession number.

Sequence Source:

The database from which the sequence used to design this probe set was taken. Examples are: GenBank®, RefSeq, UniGene, TIGR (annotations from The Institute for Genomic Research).

3. Public Domain and Genomic References

Most of the data in this section come from LocusLink and UniGene databases, and are annotations of the reference sequence on which the probe set is modeled.

Gene Symbol and Title:

A gene symbol and a short title, when one is available. Such symbols are assigned by different organizations for different species. Affymetrix annotational data come from the UniGene record. There is no indication which species-specific databank was used, but some of the possibilities include for example HUGO: The Human Genome Organization.

MapLocation:

The map location describes the chromosomal location when one is available.

Unigene_Accession:

UniGene accession number and cluster type. Cluster type can be "full length" or "est", or "---" if unknown.

LocusLink:

This information represents the LocusLink accession number.

Full Length Ref. Sequences:

Indicates the references to multiple sequences in RefSeq. The field contains the ID and description for each entry, and there can be multiple entries per probeSet.

**Example 3: Sample preparation, processing and data analysis**

Method 1:

Microarray analyses were performed utilizing the GeneChip® System (Affymetrix, Santa Clara, USA). Hybridization target preparations were performed according to recommended protocols (Affymetrix Technical Manual). In detail, at time of diagnosis, mononuclear cells were purified by Ficoll-Hypaque density centrifugation. They had been lysed immediately in RLT buffer (Qiagen, Hilden, Germany), frozen, and stored at -80°C from 1 week to 38 months. For gene expression profiling cell lysates of the leukemia samples were thawed, homogenized (QIAshredder, Qiagen), and total RNA was extracted (RNeasy Mini Kit, Qiagen). Subsequently, 5-10 µg total RNA isolated from $1 \times 10^7$ cells was used as starting material for cDNA synthesis with oligo[(dT)$_{24}$T7promotor]$_{65}$ primer (cDNA Synthesis System, Roche Applied Science, Mannheim, Germany). cDNA products were purified by phenol/chlorophorm/IAA extraction (Ambion, Austin, USA) and acetate/ethanol-precipitated overnight. For detection of the hybridized target nucleic acid biotin-labeled ribonucleotides were incorporated during the following *in vitro* transcription reaction (Enzo BioArray HighYield RNA Transcript Labeling Kit, Enzo Diagnostics). After quantification by spectrophotometric measurements and 260/280 absorbance values assessment for quality control of the purified cRNA (RNeasy Mini Kit, Qiagen), 15 µg cRNA was fragmented by alkaline treatment (200 mM Tris-acetate, pH 8.2/500 mM potassium acetate/150 mM magnesium acetate) and added to the hybridization cocktail sufficient for five hybridizations on standard GeneChip microarrays (300 µl final volume). Washing and staining of the probe arrays was performed according to the recommended Fluidics Station protocol (EukGE-WS2v4). Affymetrix Microarray Suite software (version 5.0.1) extracted fluorescence signal intensities from each feature on the microarrays as detected by confocal laser scanning according to the manufacturer's recommendations.

Expression analysis quality assessment parameters included visiual array inspection of the scanned image for the presence of image artifacts and correct grid alignment for the identification of distinct probe cells as well as both low 3'/5' ratio of housekeeping controls (mean: 1.90 for GAPDH) and high percentage of detection calls (mean: 46.3% present called genes). The 3' to 5' ratio of GAPDH probesets can be used to assess RNA sample and assay quality. Signal values of the 3' probe sets for GAPDH are compared to the Signal values of the corresponding 5' probe set. The ratio of the 3' probe set to the 5' probe set is generally no more than 3.0. A high 3' to 5' ratio may indicate degraded RNA or inefficient synthesis of ds cDNA or biotinylated cRNA (GeneChip® Expression Analysis Technical Manual, www.affymetrix.com). Detection calls are used to determine whether the transcript of a gene is detected (present) or undetected (absent) and were calculated using default parameters of the Microarray Analysis Suite MAS 5.0 software package.

Method 2:

Bone marrow (BM) aspirates are taken at the time of the initial diagnostic biopsy and remaining material is immediately lysed in RLT buffer (Qiagen), frozen and stored at -80 C until preparation for gene expression analysis.

For microarray analysis the GeneChip System (Affymetrix, Santa Clara, CA, USA) is used. The targets for GeneChip analysis are prepared according to the current Expression Analysis. Briefly, frozen lysates of the leukemia samples are thawed, homogenized (QIAshredder, Qiagen) and total RNA extracted (RNeasy Mini Kit, Qiagen).Normally 10 ug total RNA isolated from 1 x 107 cells is used as starting material in the subsequent cDNA-Synthesis using Oligo-dT-T7-Promotor Primer (cDNA synthesis Kit, Roche Molecular Biochemicals). The cDNA is purified by phenol-chlorophorm extraction and precipitated with 100% Ethanol over night. For detection of the hybridized target nucleic acid biotin-labeled ribonucleotides are incorporated during the in vitro transcription reaction (Enzo® BioArray™ HighYield™ RNA Transcript Labeling Kit, ENZO). After quantification of the purified cRNA (RNeasy Mini Kit, Qiagen), 15 ug are fragmented by alkaline treatment (200 mM Tris-acetate, pH 8.2, 500 mM potassium acetate, 150 mM magnesium acetate) and added to the hybridization cocktail sufficient for 5 hybridizations on standard GeneChip microarrays. Before expression profiling Test3 Probe Arrays (Affymetrix) are chosen for monitoring of the integrity of the cRNA. Only labeled cRNA-cocktails which showed a ratio of the messured intensity of the 3' to the 5' end of the GAPDH gene less than 3.0 are selected for subsequent hybridization on HG-U133 probe arrays (Affymetrix). Washing and staining the Probe arrays is performed as described (siehe Affymetrix-Original-Literatur (LOCKHART und LIPSHUTZ). The Affymetrix software (Microarray Suite, Version 4.0.1) extracted fluorescence intensities from each element on the arrays as detected by confocal laser scanning according to the manufacturers recommendations.

**Table 1**

| # affy | HUGO name | zres | pres | qres | Map Location |
|---|---|---|---|---|---|
| 1 233948_at | LOC255480 | 4.494575 | 6.97E-06 | 0.3131894 | 12q24.21 |
| 2 217096_at | PCLO | 4.089249 | 4.33E-05 | 0.7169452 | 7q11.23-q21.3 |
| 3 218792_s_at | FLJ20150 | 4.047751 | 5.17E-05 | 0.7169452 | 9q32 |
| 4 229259_at | GFAP | 3.998183 | 6.38E-05 | 0.7169452 | 17q21 |
| 5 217506_at | | 3.867293 | 1.10E-04 | 0.8499087 | |
| 6 218086_at | NPDC1 | 3.859751 | 1.14E-04 | 0.8499087 | 9q34.3 |
| 7 213456_at | DKFZp564D206 | 3.78748 | 1.52E-04 | 0.9309242 | 7p21.1 |
| 8 206995_x_at | SCARF1 | 3.763345 | 1.68E-04 | 0.9309242 | 17p13.3 |
| 9 219271_at | GalNac-T10 | 3.736657 | 1.86E-04 | 0.9309242 | 2p23.1 |
| 10 215506_s_at | ARHI | 3.662988 | 2.49E-04 | 0.9999846 | 1p31 |
| 11 212813_at | JAM3 | 3.562459 | 3.67E-04 | 0.9999846 | 11q25 |
| 12 226303_at | | 3.560666 | 3.70E-04 | 0.9999846 | |
| 13 243154_at | | 3.543864 | 3.94E-04 | 0.9999846 | |
| 14 211621_at | AR | 3.54073 | 3.99E-04 | 0.9999846 | Xq11.2-q12 |
| 15 212509_s_at | | 3.509968 | 4.48E-04 | 0.9999846 | |
| 16 229095_s_at | | 3.505609 | 4.56E-04 | 0.9999846 | |
| 17 210511_s_at | INHBA | 3.413141 | 6.42E-04 | 0.9999846 | 7p15-p13 |
| 18 211269_s_at | IL2RA | 3.407164 | 6.56E-04 | 0.9999846 | 10p15-p14 |
| 19 225864_at | | 3.40355 | 6.65E-04 | 0.9999846 | |
| 20 213050_at | COBL | 3.358166 | 7.85E-04 | 0.9999846 | 7p12.1 |
| 21 242406_at | | 3.353668 | 7.97E-04 | 0.9999846 | |
| 22 211841_s_at | TNFRSF25 | 3.350815 | 8.06E-04 | 0.9999846 | 1p36.2 |
| 23 207325_x_at | MAGEA1 | 3.311814 | 9.27E-04 | 0.9999846 | Xq28 |
| 24 203913_s_at | HPGD | 3.299594 | 9.68E-04 | 0.9999846 | 4q34-q35 |
| 25 207229_at | KLRA1 | 3.274672 | 1.06E-03 | 0.9999846 | 12p13-p12 |
| 26 221211_s_at | C21orf7 | 3.24633 | 1.17E-03 | 0.9999846 | 21q22.3 |
| 27 210697_at | ZNF257 | 3.241291 | 1.19E-03 | 0.9999846 | 19q13 |
| 28 218180_s_at | EPS8R2 | 3.239597 | 1.20E-03 | 0.9999846 | 11p15.5 |
| 29 244739_at | | 3.236045 | 1.21E-03 | 0.9999846 | |
| 30 216383_at | | 3.234188 | 1.22E-03 | 0.9999846 | |
| 31 208798_x_at | GOLGIN-67 | 3.222691 | 1.27E-03 | 0.9999846 | 15q11.2 |
| 32 243292_at | | 3.220777 | 1.28E-03 | 0.9999846 | |
| 33 231332_at | | 3.207364 | 1.34E-03 | 0.9999846 | |
| 34 211548_s_at | HPGD | 3.19603 | 1.39E-03 | 0.9999846 | 4q34-q35 |
| 35 236518_at | MGC15438 | 3.189706 | 1.42E-03 | 0.9999846 | 9q34.3 |
| 36 241709_s_at | DOCK1 | 3.182407 | 1.46E-03 | 0.9999846 | 10q26.13-q26.3 |
| 37 227140_at | | 3.176202 | 1.49E-03 | 0.9999846 | |
| 38 200091_s_at - HG-U133A | RPS25 | 3.174821 | 1.50E-03 | 0.9999846 | 11q23.3 |
| 39 244189_at | | 3.173329 | 1.51E-03 | 0.9999846 | |
| 40 233431_x_at | | 3.161435 | 1.57E-03 | 0.9999846 | |
| 41 206574_s_at | PTP4A3 | 3.160157 | 1.58E-03 | 0.9999846 | |
| 42 211597_s_at | HOP | 3.155641 | 1.60E-03 | 0.9999846 | 4q11-q12 |
| 43 242950_x_at | | -3.152281 | 1.62E-03 | 0.9999846 | |
| 44 204341_at | TRIM16 | 3.149655 | 1.63E-03 | 0.9999846 | 17p11.2 |
| 45 233530_at | | 3.146115 | 1.65E-03 | 0.9999846 | |

R62509EP
Table 1

| 46 200665_s_at | SPARC | 3.143607 1.67E-03 0.9999846 5q31.3-q32 |
| 47 217349_s_at | LMO6 | -3.141869 1.68E-03 0.9999846 Xp11.23-p11.22 |
| 48 204100_at | THRA | 3.140913 1.68E-03 0.9999846 17q11.2 |
| 49 236792_at | | 3.136756 1.71E-03 0.9999846 |
| 50 209386_at | TM4SF1 | 3.130134 1.75E-03 0.9999846 3q21-q25 |

zres is the test statistic of the Cox model. It is calculated as the ratio of the estimated cox regression coefficient and its estimated standard error.

P. Andersen and R. Gill. "Cox's regression model for counting processes, a large sample study", Annals of Statistics, 10:1100-1120, 1982.

Pres is non-corrected p-value
Qres is corrected p-value

| # | affy | | | | | | | |
|---|------|---|---|---|---|---|---|---|
| 1 | 233948_at | Hs.241404.0 | U47671.1 | Hs.241404.0.S1 | GenBank | Hs.363081 | 255480 | |
| 2 | 217096_at | Hs.12376.2 | AC004082 | Hs.12376.2 | GenBank | Hs.12376 | 27445 | |
| 3 | 218792_s_at | Hs.108502.0 | NM_017688.1 | g8923147 | RefSeq | Hs.108502 | 54836 | NM_017688; B-box and SPRY domain containing |
| 4 | 229259_at | Hs.286055.3 | AL133013.1 | Hs.286055.3 | GenBank | Hs.406397 | 2670 | NM_002055; glial fibrillary acidic protein |
| 5 | 217506_at | Hs.124984.0 | H49382 | Hs.124984.0_RC | GenBank | Hs.124984 | | |
| 6 | 218086_at | Hs.105547.0 | NM_015392.1 | g10181099 | RefSeq | Hs.105547 | 56654 | NM_015392; neural proliferation, differentiation and control, 1 |
| 7 | 213456_at | Hs.25956.0 | AI927000 | Hs.25956.0 | GenBank | Hs.25956 | 25928 | NM_015464; cystine knot-containing secreted protein |
| 8 | 206995_x_at | Hs.57735.0 | NM_003693.1 | g4507202 | RefSeq | Hs.57735 | 8578 | NM_003693; scavenger receptor class F, member 1 |
| 9 | 219271_at | Hs.15830.0 | NM_024572.1 | g13375743 | RefSeq | Hs.15830 | 79623 | NM_024572; UDP-GalNAc:polypeptide N-acetylgalactosaminyltransferase T10 |
| 10 | 215506_s_at | Hs.194695.1 | AK021882.1 | Hs.194695.1 | GenBank | Hs.194695 | 9077 | NM_004675; ras homolog I |
| 11 | 212813_at | Hs.55016.1 | AA149644 | Hs.55016.1.A1 | GenBank | Hs.334703 | 83700 | NM_031470; NM_032801; junctional adhesion molecule 3 precursor |
| 12 | 226303_at | Hs.46531.0 | AA706788 | Hs.46531.0 | GenBank | Hs.46531 | | |
| 13 | 243154_at | Hs.86650.0 | AA215381 | Hs.86650.0.A1 | GenBank | Hs.86650 | | |
| 14 | 211621_at | g178655 | M73069.1 | g178655 | GenBank | Hs.99915 | 367 | NM_000044; androgen receptor |
| 15 | 212509_s_at | Hs.250723.2 | BF968134 | Hs.250723.2.S1 | GenBank | Hs.356623 | | |
| 16 | 229095_s_at | Hs.93701.0 | AI797263 | Hs.93701.0.S1 | GenBank | Hs.93701 | | |
| 17 | 210511_s_at | Hs.727.1 | M13436.1 | g186414 | GenBank | Hs.727 | 3624 | NM_002192; inhibin beta A subunit precursor |
| 18 | 211269_s_at | Hs.1724.1 | K03122.1 | g186319 | GenBank | Hs.1724 | 3559 | NM_000417; interleukin 2 receptor, alpha chain precursor |
| 19 | 225864_at | Hs.49136.0 | AL039862 | Hs.49136.0.A1 | GenBank | Hs.49136 | | |
| 20 | 213050_at | Hs.33010.0 | AA594937 | Hs.33010.0.S1 | GenBank | Hs.33010 | 23242 | NM_015198; KIAA0633 protein |
| 21 | 242406_at | Hs.163242.0 | AI870547 | Hs.163242.0.A1 | GenBank | Hs.163242 | | |
| 22 | 211841_s_at | Hs.180338.9 | U94510.1 | g2071966 | GenBank | Hs.180338 | 8718 | NM_003790; tumor necrosis factor receptor superfamily, member 25 isoform 2 precursor NM_148965; tumor necrosis factor receptor superfamily, member 25 isoform 1 precursor NM_148966; tumor necrosis factor receptor superfamily, member 25 isoform 3 precursor NM_148967; tumor necrosis factor receptor superfamily, member 25 isoform 4 precursor NM_148968; tumor necrosis factor receptor superfamily, member 25 isoform 5 precursor NM_148969; tumor necrosis factor receptor superfamily, member 25 isoform 6 precursor |

EP 1 533 618 A1

NM_148970; tumor necrosis factor receptor superfamily, member 25 isoform 7 precursor NM_148971; tumor necrosis factor receptor superfamily, member 25 isoform 8 precursor NM_148972; tumor necrosis factor receptor superfamily, member 25 isoform 9 precursor NM_148973; tumor necrosis factor receptor superfamily, member 25 isoform 10 precursor NM_148974; tumor necrosis factor receptor superfamily, member 25 isoform 11 precursor

| 23 | 207325_x_at | Hs.72879.0 | NM_004988.1 | g4826821 | RefSeq | Hs.72879 | 4100 | NM_004988; melanoma antigen, family A, 1 |
|----|-------------|------------|-------------|----------|---------|----------|------|------------------------------------------|
| 24 | 203913_s_at | Hs.77348.0 | AL574184 | Hs.77348.0 | GenBank | Hs.77348 | 3248 | NM_000860; hydroxyprostaglandin dehydrogenase 15-(NAD) |
| 25 | 207229_at | Hs.159297.0 | NM_006611.1 | g5729898 | RefSeq | Hs.159297 | 10748 | NM_006611; killer cell lectin-like receptor subfamily A, member 1 |
| 26 | 221211_s_at | Hs.41267.0 | NM_020152.1 | g9910145 | RefSeq | Hs.41267 | 56911 | NM_020152; chromosome 21 open reading frame 7 |
| 27 | 210697_at | Hs.283900.0 | AF070651.1 | g4454677 | GenBank | Hs.283900 | 113835 | NM_033468; zinc finger protein 257 |
| 28 | 218180_s_at | Hs.55016.0 | NM_022772.1 | g12232452 | RefSeq | Hs.55016 | 64787 | NM_022772; epidermal growth factor receptor pathway substrate 8-like protein 2 |
| 29 | 244739_at | Hs.96297.0 | AI051769 | Hs.96297.0.A1 | GenBank | Hs.96297 | | |
| 30 | 216383_at | Hs.283952.0 | U52111 | Hs.283952.0 | GenBank | | | |
| 31 | 208798_x_at | Hs.182982.0 | AF204231.1 | g6808610 | GenBank | Hs.182982 | 23015 | NM_015003; golgin-67 isoform a NM_181076; golgin-67 isoform b NM_181077; golgin-67 isoform c |
| 32 | 243292_at | Hs.197613.0 | BF589994 | Hs.197613.0_RC | GenBank | Hs.197613 | | |
| 33 | 231332_at | Hs.254986.0 | AW295037 | Hs.254986.0.A1 | GenBank | Hs.254986 | | |
| 34 | 211548_s_at | Hs.77348.1 | J05594.1 | g1203981 | GenBank | Hs.77348 | 3248 | NM_000860; hydroxyprostaglandin dehydrogenase 15-(NAD) |
| 35 | 236518_at | Hs.129544.0 | BE208843 | Hs.129544.0_RC | GenBank | Hs.283506 | 84960 | NM_032874; hypothetical protein MGC15438 |
| 36 | 241709_s_at | Hs.309770.0 | AA599017 | Hs.309770.0.A1 | GenBank | Hs.82295 | 1793 | NM_001380; dedicator of cyto-kinesis 1 |
| 37 | 227140_at | Hs.28792.0 | AI343467 | Hs.28792.0 | GenBank | Hs.28792 | | |
| 38 | 200091_s_at - HG-U133A | Hs.113029.1 | AA888388 | Hs.113029.1.A1 | GenBank | Hs.409158 | 6230 | NM_001028; ribosomal protein S25 |
| 39 | 244189_at | Hs.263414.0 | AI888657 | Hs.263414.0.A1 | GenBank | Hs.348657 | | |
| 40 | 233431_x_at | Hs.287527.0 | AU148142 | Hs.287527.0.S1 | GenBank | Hs.287527 | | |
| 41 | 206574_s_at | Hs.43666.0 | NM_007079.1 | g6857821 | RefSeq | Hs.43666 | 11156 | NM_007079; protein tyrosine phosphatase type IVA, member 3 isoform 2 NM_032611; protein tyrosine phosphatase type IVA, member 3 isoform 1 |
| 42 | 211597_s_at | g13560280 | AB059408.1 | g13560280 | GenBank | Hs.13775 | 84525 | NM_032495; homeodomain-only protein NM_139211; homeodomain-only protein NM_139212; homeodomain-only protein |

| | | | | | | | |
|---|---|---|---|---|---|---|---|
| 43 | 242950_x_at | Hs.291236.0 | AW969652 | Hs.291236.0_RC | GenBank | Hs.291236 | |
| 44 | 204341_at | Hs.241305.0 | NM_006470.1 | g5453643 | RefSeq | Hs.241305 | 10626 NM_006470; tripartite motif-containing 16 |
| 45 | 233530_at | Hs.306949.0 | W26305 | Hs.306949.0.S1 | GenBank | | |
| 46 | 200665_s_at | Hs.111779.0 | NM_003118.1 | g4507170 | RefSeq | Hs.111779 | 6678 NM_003118; secreted protein, acidic, cysteine-rich (osteonectin) |
| 47 | 217349_s_at | Hs.166173.1 | U93305 | Hs.166173.1 | GenBank | Hs.166173 | 4007 NM_006150; LIM domain only 6 |
| 48 | 204100_at | Hs.724.0 | NM_003250.1 | g4507494 | RefSeq | Hs.724 | 7067 NM_003250; thyroid hormone receptor, alpha (erythroblastic leukemia viral (v-erb-a) oncogene homolog, avian) |
| 49 | 236792_at | Hs.121449.0 | AA813320 | Hs.121449.0.A1 | GenBank | Hs.121449 | |
| 50 | 209386_at | Hs.3337.0 | AI346835 | Hs.3337.0.A2 | GenBank | Hs.351316 | 4071 NM_014220; transmembrane 4 superfamily member 1 |

EP 1 533 618 A1

**Claims**

1. A method for distinguishing prognostically definable AML subtypes with normal karyotype into different prognosis subsets in a sample, the method comprising determining the expression level of markers selected from the markers identifiable by their Affymetrix Identification Numbers (affy id) as defined in Table 1,

wherein

a high expression of at least one polynucleotide defined by any of the numbers 1, 2, 3, 4, 5, 6, 7, 8, 9, 10, 11, 12, 13, 14, 15, 16, 17, 19, 20, 21, 22, 23, 24, 25, 26, 27, 28, 29, 30, 31, 32, 33, 34, 35, 36, 37, 38, 39, 40, 42, 43, 44, 46, 47, 48, 49, and/or 50 of Table 1,

is indicative for median event-free survival (EFS).

2. The method according to claim 1 wherein the polynucleotide is labelled.

3. The method according to claim 1 or 2, wherein the label is a luminescent, preferably a fluorescent label, an enzymatic or a radioactive label.

4. The method according at least one of the claims 1-3, wherein the expression level of at least two, preferably of at least ten, more preferably of at least 25, most preferably of 50 of the markers of at least one of the Table 1 is determined.

5. The method according to at least one of the claims 1-4, wherein the expression level of markers expressed lower in a first subtype than in at least one second subtype, which differs from the first subtype, is at least 5 %, 10% or 20%, more preferred at least 50% or may even be 75% or 100%, i.e. 2-fold lower, preferably at least 10-fold, more preferably at least 50-fold, and most preferably at least 100-fold lower in the first subtype.

6. The method according to at least one of the claims 1-4, wherein the expression level of markers expressed higher in a first subtype than in at least one second subtype, which differs from the first subtype, is at least 5 %, 10% or 20%, more preferred at least 50% or may even be 75% or 100%, i.e. 2-fold higher, preferably at least 10-fold, more preferably at least 50-fold, and most preferably at least 100-fold higher in the first subtype.

7. The method according to at least one of the claims 1-6, wherein the sample is from an individual having AML.

8. The method according to at least one of the claims 1-7, wherein at least one polynucleotide is in the form of a transcribed polynucleotide, or a portion thereof.

9. The method according to claim 8, wherein the transcribed polynucleotide is a mRNA or a cDNA.

10. The method according to claim 8 or 9, wherein the determining of the expression level comprises hybridizing the transcribed polynucleotide to a complementary polynucleotide, or a portion thereof, under stringent hybridization conditions.

11. The method according to at least one of the claims 1-7, wherein at least one polynucleotide is in the form of a polypeptide, or a portion thereof.

12. The method according to at least one of claims 8, 9 or 12, wherein the determining of the expression level comprises contacting the polynucleotide or the polypeptide with a compound specifically binding to the polynucleotide or the polypeptide.

13. The method according to claim 12, wherein the compound is an antibody, or a fragment thereof.

14. The method according to at least one of the claims 1-13, wherein the method is carried out on an array.

15. The method according to at least one of the claims 1-14, wherein the method is carried out in a robotics system.

16. The method according to at least one of the claims 1-15, wherein the method is carried out using microfluidics.

17. Use of at least one marker as defined in at least one of the claims 1-3 for the manufacturing of a diagnostic for distinguishing prognostically definable AML subtypes with normal karyotype.

18. The use according to claim 17 for distinguishing prognostically definable AML subtypes with normal karyotype into different prognosis subsets in an individual having AML.

19. A diagnostic kit containing at least one marker as defined in at least one of the claims 1-3 for distinguishing prognostically definable AML subtypes with normal karyotype, in combination with suitable auxiliaries.

20. The diagnostic kit according to claim 19, wherein the kit contains a reference for the prognostically definable AML subtypes with normal karyotype.

21. The diagnostic kit according to claim 20, wherein the reference is a sample or a data bank.

22. An apparatus for distinguishing prognostically definable AML subtypes with normal karyotype into different prognosis subsets in a sample containing a reference data bank.

23. The apparatus according to claim 22, wherein the reference data bank is obtainable by comprising

   (a) compiling a gene expression profile of a patient sample by determining the expression level of at least one marker selected from the markers identifiable by their Affymetrix Identification Numbers (affy id) as defined in Table 1, and
   (b) classifying the gene expression profile by means of a machine learning algorithm.

24. The apparatus according to claim 23, wherein the machine learning algorithm is selected from the group consisting of Weighted Voting, K-Nearest Neighbors, Decision Tree Induction, Support Vector Machines, and Feed-Forward Neural Networks, preferably Support Vector Machines.

25. The apparatus according to at least one of the claims 22-24, wherein the apparatus contains a control panel and/ or a monitor.

26. A reference data bank for distinguishing prognostically definable AML subtypes with normal karyotype into different prognosis subsetsobtainable by comprising

   (a) compiling a gene expression profile of a patient sample by determining the expression level of at least one marker selected from the markers identifiable by their Affymetrix Identification Numbers (affy id) as defined in Table 1 and
   (b) classifying the gene expression profile by means of a machine learning algorithm.

27. The reference data bank according to claim 26, wherein the reference data bank is backed up and/or contained in a computational memory chip.

**European Patent**

**Office**

## PARTIAL EUROPEAN SEARCH REPORT

which under Rule 45 of the European Patent Convention
shall be considered, for the purposes of subsequent
proceedings, as the European search report

Application Number

EP 03 02 5338

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | US 2003/011943 A1 (GILL HARDAYAL SINGH ET AL) 16 January 2003 (2003-01-16) * the whole document * in particular Examples 4 and 5. | 1-27 | G01N33/574 C12Q1/68 |
| Y | WO 03/083140 A (WONG LIMSOON ;YEOH ENG-JUH (SG); DOWNING JAMES R (US); WILKINS DAW) 9 October 2003 (2003-10-09) * the whole document * in particular pages 5-14. | 1-27 | |
| Y | WO 03/039443 A (DEUTSCHES KREBSFORSCH ;HAFERLACH TORSTEN (DE); EILS ROLAND (DE); K) 15 May 2003 (2003-05-15) * the whole document * | 1-27 | |
| Y | KOHLMANN A ET AL: "MOLECULAR CHARACTERIZATION OF ACUTE LEUKEMIAS BY USE OF MICROARRAY TECHNOLOGY" GENES, CHROMOSOMES & CANCER, XX, XX, vol. 37, no. 4, August 2003 (2003-08), pages 396-405, XP008025253 * the whole document * | 1-27 | **TECHNICAL FIELDS SEARCHED** (Int.Cl.7) G01N C12Q |

-/--

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 2 March 2004 | Thumb, W |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent
Office

**PARTIAL EUROPEAN SEARCH REPORT**

Application Number

EP 03 02 5338

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| Y | EP 1 043 676 A (WHITEHEAD BIOMEDICAL INST) 11 October 2000 (2000-10-11) * the whole document * ----- | 1-27 | |
| Y,D | GOLUB T R ET AL: "Molecular classification of cancer: Class discovery and class prediction by gene expression monitoring" SCIENCE, AMERICAN ASSOCIATION FOR THE ADVANCEMENT OF SCIENCE,, US, vol. 286, no. 5439, 15 October 1999 (1999-10-15), pages 531-537, XP002207658 ISSN: 0036-8075 * the whole document * ----- | 1-27 | TECHNICAL FIELDS SEARCHED (Int.Cl.7) |
| Y | DUGAS M ET AL: "A comprehensive leukemia database: integration of cytogenetics, molecular genetics and microarray data with clinical information, cytomorphology and immunophenotyping" LEUKEMIA, MACMILLAN PRESS LTD, US, vol. 15, no. 12, December 2001 (2001-12), pages 1805-1810, XP002263731 ISSN: 0887-6924 * the whole document * ----- | 1-27 | |
| Y | DUGAS MARTIN ET AL: "Impact of integrating clinical and genetic information." IN SILICO BIOLOGY, vol. 2, no. 3, 2002, pages 383-391, XP001179418 ISSN: 1386-6338 (ISSN print) * the whole document * ----- -/-- | 1-27 | |

EPO FORM 1503 03.82 (P04C10)

## European Patent Office

### PARTIAL EUROPEAN SEARCH REPORT

Application Number

EP 03 02 5338

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| A | ALIZADEH A ET AL: "THE LYMPHOCHIP: A SPECIALIZED CDNA MICROARRAY FOR THE GENOMIC-SCALE ANALYSIS OF GENE EXPRESSION IN NORMAL AND MALIGNANT LYMPHOCYTES" COLD SPRING HARBOR SYMPOSIA ON QUANTITATIVE BIOLOGY, BIOLOGICAL LABORATORY, COLD SPRING HARBOR, NY, US, vol. 64, no. 1, 1999, pages 71-78, XP001099007 ISSN: 0091-7451 * the whole document * | 1-27 | |
| A | SCHNITTGER S ET AL: "Screening for MLL tandem duplication in 387 unselected patients with AML identify a prognostically unfavorable subset of AML" LEUKEMIA (BASINGSTOKE), vol. 14, no. 5, May 2000 (2000-05), pages 796-804, XP002272116 ISSN: 0887-6924 * abstract * | 1-27 | TECHNICAL FIELDS SEARCHED (Int.Cl.7) |
| A | BOISSEL N ET AL: "Prognostic significance of FLT3 internal tandem repeat in patients with de novo acute myeloid leukemia treated with reinforced courses of chemotherapy" LEUKEMIA (BASINGSTOKE), vol. 16, no. 9, September 2002 (2002-09), pages 1699-1704, XP002272117 ISSN: 0887-6924 * abstract * | 1-27 | |

EPO FORM 1503 03.82 (P04C10)

**European Patent Office**

**INCOMPLETE SEARCH
SHEET C**

Application Number

EP 03 02 5338

Claim(s) searched incompletely:
    22-27

Reason for the limitation of the search (non-patentable invention(s)):

Article 52 (2)(d) EPC – Presentation of information

The claims were only searched with regards to the underlying method of generating a reference data base for distinguishing prognistically definable AML subtypes.

European Patent
Office

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-27 (partially)

| European Patent Office | LACK OF UNITY OF INVENTION SHEET B | Application Number EP 03 02 5338 |

The Search Division considers that the present European patent application does not comply with the requirements of unity of invention and relates to several inventions or groups of inventions, namely:

1. claims: 1-27 (partially)

   A method for distinguishing definable AML subtypes with normal karyotpye into different prognosis subsets, the method comprising determining the expression of LOC255480. A diagnostic kit containing said marker and an apparatus comprising a reference data bank, wherein the reference data bank is obtainable by determining the expression level of LOC255480.

   ---

2. claims: 1-27 (all partially)

   Inventions 2-50
   Methods for distinguishing definable AML subtypes with normal karyotpye into different prognosis subsets, the method comprising determining individually the expression level of the markers listed in table 1, positions 2-50. Use of said markers for the manufacture of diagnostics. Diagnostic kits containing said markers and apparatus comprising a reference data bank, wherein the reference data bank is obtainable by determining the expression levels of said markers.

   ---

## ANNEX TO THE EUROPEAN SEARCH REPORT
## ON EUROPEAN PATENT APPLICATION NO.

EP 03 02 5338

This annex lists the patent family members relating to the patent documents cited in the above-mentioned European search report.
The members are as contained in the European Patent Office EDP file on
The European Patent Office is in no way liable for these particulars which are merely given for the purpose of information.

02-03-2004

| Patent document cited in search report | | Publication date | Patent family member(s) | | Publication date |
|---|---|---|---|---|---|
| US 2003011943 | A1 | 16-01-2003 | NONE | | |
| WO 03083140 | A | 09-10-2003 | WO US | 03083140 A2 2004018513 A1 | 09-10-2003 29-01-2004 |
| WO 03039443 | A | 15-05-2003 | EP WO | 1308522 A1 03039443 A2 | 07-05-2003 15-05-2003 |
| EP 1043676 | A | 11-10-2000 | CA EP JP US US US | 2304876 A1 1043676 A2 2001017171 A 2003017481 A1 6647341 B1 2003073083 A1 | 09-10-2000 11-10-2000 23-01-2001 23-01-2003 11-11-2003 17-04-2003 |

For more details about this annex : see Official Journal of the European Patent Office, No. 12/82